(19) **Europäisches Patentamt / European Patent Office / Office européen des brevets**

(11) **EP 4 481 058 A1**

(12) # EUROPEAN PATENT APPLICATION

(43) Date of publication:
**25.12.2024   Bulletin 2024/52**

(21) Application number: **23181242.1**

(22) Date of filing: **23.06.2023**

(51) International Patent Classification (IPC):
**C12Q 1/6818** (2018.01)        **C12Q 1/6851** (2018.01)
**G01F 11/00** (2006.01)        **G01N 21/00** (2006.01)

(52) Cooperative Patent Classification (CPC):
(C-Sets available)
**C12Q 1/6851; C12Q 1/6818; G01N 21/6428;**
G01N 2021/6421; G01N 2021/6441        (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **QIAGEN GmbH**
**40724 Hilden (DE)**

(72) Inventors:
• **LÜERSSEN, Dietrich**
  **08037 Barcelona (ES)**

• **MÉRIDA FERNÁNDEZ, José**
  **08018 Barcelona (ES)**
• **SÁNCHEZ LAORDERN, Celia**
  **08830 Sant Boi de Llobregat (ES)**
• **PAU PARRA, Lucia**
  **22500 Bifenar (Huesca) (ES)**
• **MOLINS GALVEZ, Albert**
  **08018 Barcelona (ES)**
• **JUNYENT MUÑOZ, Joan**
  **08028 Barcelona (ES)**

(74) Representative: **König Szynka Tilmann von Renesse**
**Patentanwälte Partnerschaft mbB Düsseldorf**
**Mönchenwerther Straße 11**
**40545 Düsseldorf (DE)**

(54) **METHOD, USE AND DEVICE FOR SIGNAL COLLECTION IN A PROCESS UTILIZING FLUORESCENCE AT DIFFERENT WAVELENGTHS**

(57)     The invention refers to a method for signal collection in a process that utilizes fluorescence detection at multiple wavelengths, especially a qPCR process. The method uses at least two channels, wherein for each of the at least two channels an excitation condition and a detection condition is provided for one of the multiple wavelengths, respectively, and wherein during the signal collection in the fluorescence detection: i) a first signal in the first channel is recorded; ii) a second signal in the second channel is recorded; and iii) a crosstalk correlation is determined using a dependency between the first signal and the second signal of the fluorescence detection.

**EP 4 481 058 A1**

(52) Cooperative Patent Classification (CPC): (Cont.)

C-Sets
**C12Q 1/6818, C12Q 2537/143, C12Q 2537/165;**
**C12Q 1/6851, C12Q 2537/143, C12Q 2537/165**

**Description**

**FIELD OF THE INVENTION**

The invention refers to a method for signal collection in a process that utilizes fluorescence detection at multiple wavelengths, especially a qPCR process, using at least two channels, wherein for each of the at least two channels an excitation condition and a detection condition is provided for one of the multiple wavelengths, respectively, and wherein during the signal collection in the fluorescence detection: i) a first signal in the first channel is recorded; and ii) a second signal in the second channel is recorded. Further, the invention refers to a use of signal collection in a process that utilizes fluorescence detection at multiple wavelengths, especially, a qPCR process, wherein in the process at least two channels are used, and each signal for the two channels is recorded. Moreover, the invention refers to a device for performing a process that utilizes fluorescence detection at multiple wavelengths, especially a qPCR or dPCR process, using at least two channels.

**BACKGROUND OF THE INVENTION**

Nucleic acid amplification can be used for several purposes. One of the purposes is to analyze nucleic acids including, for example, DNA and RNA. Types of nucleic acid amplification include polymerase chain reaction (PCR), transcription mediated amplification (TMA), ligase chain reaction (LCR), strand-displacement amplification (SDA), loop-mediated isothermal amplification (LAMP) and nucleic acid sequence based amplification (NASBA).

PCR in general amplifies a specific region of a DNA strand (the DNA target). Typically, PCR consists of a series of a plurality of repeated temperature changes, called thermal cycles, with each cycle commonly consisting of three discrete temperature steps: denaturation, annealing, and extension. During the denaturation. a liquid sample is heated at approximately 94° C. During this process double DNA strands "melt" open into single stranded DNA and all enzymatic reactions stop. During annealing, the single stranded DNA is cooled to approximately 54°C. At this temperature, primers bind or "anneal" to the ends of the DNA strands. During extension, the sample is heated to approximately 75°C. At this temperature, nucleotides add to the primers and eventually a complementary copy of the DNA template is formed.

Quantitative PCR (qPCR) is used to measure the quantity of a target sequence (commonly in real-time). It quantitatively measures starting amounts of DNA, cDNA, or RNA. Quantitative PCR is commonly used to determine whether a DNA sequence is present in a sample and the number of its copies in the sample. Quantitative PCR has a very high degree of precision. Quantitative PCR methods can make use of fluorescent dyes, such as Sybr Green, EvaGreen or Fluorophore-containing DNA probes, such as TaqMan, to measure the amount of amplified product in real time. Further, it may be possible to make use of a molecular beacon which is a single-stranded bi-labeled fluorescent probe held in a hairpin-loop confirmation by complementary stem sequences at both ends of the probe. Quantitative PCR is also sometimes abbreviated to real-time PCR.

For each of the fluorescent dyes or fluorophore-containing DNA probes, an optical module can be provided which can form at least part of an "optical channel" for optical detection of the fluorescent dyes or fluorophore-containing DNA probes. A system which is able to detect multiple fluorescent dyes or fluorophore-containing DNA probes may be referred to as a multiplex system in which multiple qPCR reactions can take place in parallel.

The optical module can be selected to maximize sensitivity and minimize the amount of spectral crosstalk, which is carry-over signals from one dye on an optical module that is configured to detect spectral signals from another dye. Therefore, in multiplex qPCR reactions, spectral crosstalk between different optical channels is mostly unavoidable even if each of the optical modules is optimized for detection of a respective fluorescent dye or fluorophore-containing DNA probe at a discrete wavelength band. This crosstalk has traditionally been characterised once (e.g., for a given instrument, or for an instrument class), and then corrected for each subsequent experiment with this fixed value.

US 2018/0201987 A1 discloses a compensation for spectral crosstalk in multiplex nucleic acid amplification. A set of crosstalk correction factors is determined from a calibration process. The calibration process is performed using actual real-time amplification data obtained with the appropriate probes in a series of single-plex reactions. All channels are scanned for each reaction even though only one target is present, to quantify the amount of crosstalk of that probe onto the neighboring modules.

It has been found that the prior consideration of crosstalk works well in many situations, but in some cases there was still a crosstalk which could not be handled with.

It is an object of the invention to overcome drawbacks of the prior consideration of crosstalk, especially the correction of crosstalk. Especially, the consideration regarding the crosstalk influence can be made more accurate, faster and/or less recources consuming.I

## SUMMARY OF THE INVENTION

According to the invention it has been surprisingly found that the crosstalk can be considered by looking at the cycle measurement or amplification signal itself, i.e. without a need to perform a (special) calibration process. According to the invention, the crosstalk is considered between the signal of the channels per qPCR experiment.

It is assumed that a correlation between the channels can be evaluated from the signal of the channels itself. Therefore, the invention has broken with the preconceived and fixed idea that a calibration process has to be performed in addition to the measurement or amplification process that is being measured during the "real" amplification process.

According to the invention, a crosstalk can be considered even if there is a (small) shift in the emission wavelength of the chosen fluorescent dyes or fluorophore-containing DNA probes. This (small shift) can be provoked by different salt concentrations, pH value, and even isomers of the dyes which can have different emission characteristics and a wavelength shift. As a result, according to the invention, a crosstalk can be considered even for those cases in which prior to the invention there was a potential of introducing erroneous evaluation of values, and in some cases also the erroneous calling of positive or negative results (under- and over-compensation of the crosstalk). Thus, whereas the prior art considers a static crosstalk influence when performing a calibration process, the invention considers a dynamic crosstalk. This makes it possible to provide a method to correct for the influence of crosstalk for each sample or reaction chamber alone. While this may sound more difficult at first, further improvement is possible through sample and/or reaction chamber dependent correction of crosstalk. Since the data of the measurement are also used as correction for the crosstalk - and no additional measurements - the procedure is simpler.

According to a first aspect, a method for signal collection in a process that utilizes fluorescence detection at multiple wavelengths, especially a qPCR process, is provided. According to the method at least two channels are used, wherein for each of the at least two channels an excitation condition and a detection condition is provided for one wavelength band out of a plurality of wavelength bands, respectively, and wherein during the signal collection in the fluorescence detection: i) a first signal in the first channel is recorded; ii) a second signal in the second channel is recorded; and iii) a crosstalk correlation is determined using a dependency between the first signal and the second signal of the fluorescence detection.

For brevity, and without loss of generality, we may substitute the term "one of a plurality of wavelength bands" by the term "one of a plurality of wavelengths". According to the description, the term "plurality" encompasses two, three or even more.

The method is particularly advantageous because no calibration process or calibration measurement has to be performed. A change of the crosstalk correlation can be considered directly from the measurement itself.

One of the applications of the present invention is qPCR (real-time PCR). However, the invention can be extended to other processes, which utilize fluorescence detection at multiple wavelengths. The processes which are especially appropriate to make use of the invention are digital PCR, digital droplet PCR, Next Generation Sequencing (NGS), and fluorescence microscopy.

The term "signal" in the description, especially each of the terms "first signal" and "second signal", is a signal determined in a "real measurement process", which means that at least one of the first and the second signal is a signal that is to be corrected by the crosstalk correlation determined by the signals. The term "signal" encompasses a signal obtained from a probe under investigation for quantitative measurement. The term "signal" encompasses an amplification curve measured from a probe under investigation, especially a probe to be measured by a qPCR.

Digital PCR (dPCR), and digital droplet PCR (ddPCR) are methods that make use of an endpoint fluorescence, especially in small reaction vessels, that ideally contain only a single template molecule to start with. Thus, an assumption can be made regarding dPCR and ddPCR that most vessels contain only a single target. Therefore, according to the core idea, in each reaction vessel the channel crosstalk can be evaluated not via the amplification curve, but via the assumption that most vessels contain only a single target. In these vessels, the comparison between the first and the second signal is used for determination of the (dynamic) crosstalk. The (dynamic) crosstalk according to the core idea of the invention can be applied to the vessels which will contain two or more targets. Therefore, the (dynamic) crosstalk according to the core idea of the invention can be applied to vessels or reaction chambers for which at least two signals (two channels) are detected. The signals to be corrected show a not flattened curve and therefore indicate that the vessel or reaction chamber contains two or more targets. The provided dynamic crosstalk correction can help improve signal-to-noise ratio. Therefore, the term "signal" is not limited to an amplification curve but refers in general to a signal determining the quantity or quality or value obtained for a probe under investigation regarding the respective measurement method. The two signals (or at least one of them) can be corrected by the crosstalk correlation obtained from the two signals itself.

The term "fluorescence detection" encompasses using a detection of fluorescence at a wavelength or wavelength band by a detector, wherein the fluorescence measured, for example fluorescence intensity, is a measure of the number of a nucleic acid sequence present in a probe, for example the number of double-stranded DNA. For the particular useful process of qPCR, fluorescence is used to measure the amplification. Despite the fact that in general different techniques can be used, each of the techniques utilizes a fluorescence intensity which depends on the amplified respective target concentration. The fluorescence may originate from a fluorescent dye or fluorophore, whose fluorescence may increase upon binding to a specific probe or to DNA. Furthermore, in an alternative or additional measure, the fluorescence may originate from a fluorescently labeled probe whose fluorescence changes when bound to the amplified target sequence. Moreover, a fluorescence signal may be determined when the probe is made fluorescently active.

In qPCR, a data acquisition device measures an output signal of a detector associated with the respective wavelength of the channel to observe the amplification in real time.

The term "signal collection" in the process encompasses to determine a signal in the at least two channels over a plurality of "excitation and detection periods", wherein an "excitation and detection period" encompasses a time point or time period on or over which fluorescence intensity is measured. For example in qPCR, an "excitation and detection period" is a thermal amplification cycle. The signal collection can be, but does not need to be, performed until saturation of fluorescence intensity is reached in at least one of the two channels.

The term "channel" encompasses an "optical channel" which comprises an optical module for optical detection of a wavelength or a wavelength band. Thus, each channel is capable of detecting a wavelength associated with the fluorescence which has to be determined, for example the fluorescence signal/intensity of a fluorescent dye or fluorophore.

The terms "first signal" and "second signal" encompass to collect or determine the fluorescence signal in the at least two channels and to obtain a fluorescence, for example the fluorescence intensity or a value relating to the fluorescence intensity, over the number of cycles. The crosstalk correction can be performed for each of the amplification processes, i.e. probes and/or reaction chambers.

A crosstalk correlation is determined using a dependency between the first signal and the second signal of the fluorescence detection. That is, the crosstalk correlation depends on the signals obtained when performing the process itself. The crosstalk correlation can only refer to the data measurement during the cycles. No further input in form of a crosstalk calibration is necessary.

The crosstalk correlation can be used as a correction. With this regard, the term "crosstalk correlation" can also be interpreted as a value that can be used for the correction of crosstalk.

Therefore, crosstalk correlation is determined using a dependency between the first signal and the second signal of the fluorescence detection. That is, the crosstalk correlation depends on the signals obtained when performing the process itself. The crosstalk correlation refers only to the measurement of end point fluorescence in multiple independent reaction vessels. No further input in form of a crosstalk calibration is necessary.

In accordance with the invention, the fluorescence in the channels can be detected in parallel, however, it is also possible to detect the fluorescence in the channels one after the other.

In a preferred embodiment, a linear dependency between the first signal and the second signal of the process is considered. Therefore, it may be considered that a linear crosstalk correlation is present. It has been surprisingly found that this correlation can be considered even for the signal of the measurement itself.

In a preferred embodiment, a linear regression between the at least two channels is carried out. Any suitable linear regression can be carried out, to fit a straight line to the function of the signals of the first channel over the signal of the second channel or vice versa.

In a preferred embodiment, the linear regression is performed between a range of cycles that is specified by the cycle threshold Ct and the symmetry midpoint $C_{INF}$ of a sigmoidal curve in the signal of the channels. The linear regression is performed between Ct and $C_{INF} + \Delta_{cycle}$, wherein $\Delta_{cycle} = C_{INF} - Ct$.

In a preferred embodiment, especially when more than two signals are considered, at least one further linear regression is carried out for at least one additional adjacent channel of one of the at least two channels. The further linear regression(s) can be carried out in the same manner as the linear regression between the at least two channels.

The term "adjacent" regarding channels encompasses that two channels are adjacent to each other if the peak fluorescences (wavelength or wavelength band of the channel to be detected) of the two channels are separated by a distance less than a maximum separation threshold and/or if the wavelength bands overlap at some point.

In a preferred embodiment, especially for a qPCR process, for each of the linear regressions a crosstalk value $c_v$ depending on the slope of the linear regression and a goodness $R^2$ are obtained. A quality or goodness of the crosstalk dependency can be obtained with simple methods.

The term "depending on" encompasses that the crosstalk value $c_v$ can be the slope of the linear regression or a value substantially equal to the slope. The term "substantially equal to the slope" encompasses that the value of the slope is defined by the regression, however, small deviations might be taken into account which are in the limits defined by at least one error bar. The term "error bar" previously used encompasses an uncertainty of the slope during a fitting process. Therefore, the slope is considered to be the value of the crosstalk value $c_v$ within one, two or more error bars.

In a preferred embodiment, it is decided whether a linear regression is considered valid from an automatic supported or autonomously automatic manner. It can be provided that a very intelligent crosstalk consideration can be performed in an automatic manner without a special calibration process, just by considering the measurement data itself.

In a preferred embodiment, the consideration whether the linear regression is considered valid is carried out on a decision whether the crosstalk value $c_v$ is above a maximum boundary value. This criterion is quite useful and very simple to look for. The crosstalk consideration is very easy to obtain.

In a preferred embodiment, the consideration whether the linear regression is considered valid is carried out on a decision whether the crosstalk value $c_v$ is below a minimum boundary value. This criterion is quite useful and very simple to look for. The crosstalk consideration is very easy to obtain.

In a preferred embodiment, the consideration whether the linear regression is considered valid is carried out on a decision whether their goodness $R^2$ is above a certain value. This criterion is quite useful and very simple to look for. The crosstalk consideration is very easy to obtain.

In a preferred embodiment, a correction is performed by calculating the value of the signal value of one of the at least two channels minus the slope of a linear regression between the at least two channels multiplied by the value of the second channel of the at least two channels.

In a preferred embodiment, the at least two channels are adjacent channels.

In a preferred embodiment, the signal in the two channels is recorded until saturation of the signal is detected. Therefore, a whole measurement can be performed. The signals of the measurement in the at least two channels can be considered as a whole.

In a preferred embodiment, especially for a qPCR process, the recorded signal for each of the at least two channels is fitted. Thus, a reliable optimized function can be considered. In case a fitting is performed for the at least two channels, the possibility of outliers and/or the influence of outliers can be reduced. Thus, for determining the crosstalk influence and calculate the crosstalk correction parameters can be obtained from the fitting function. To perform the fitting, initial measurement, i.e. especially for initial cycles, and/or outliers can be discarded.

In a preferred embodiment, the initial cycles that may be discarded can be set in a configuration file by an expert and aim to remove the cycles where the baseline is not stable. The outlier's identification can be based on several parameters set in the configuration file. A LOESS regression with degree 1 polynomial is applied to the available data points of a channel. The data points with largest absolute residuals can be candidate outliers. If the absolute residual of this candidate is bigger than the threshold, this point can be flagged as outlier. This process can be repeated until the absolute residuals are below the threshold or the maximum allowed number of outliers is reached.

For the fitting, the following logistic function may be used:

$$F(c) = \alpha c + \beta + \frac{a}{\left(1 + \left(\frac{c}{c_0}\right)^b\right)}$$

An algorithm for curve fitting can be used which is called BOBYQA. BOBYQA is an acronym for Bound Optimization BY Quadratic Approximation. More details regarding the BOBYQA algorithm can be found in Powell, M. J. D., June 2009, "The BOBYQA algorithm for bound constrained optimization without derivatives (Report)", Department of Applied Mathematics and Theoretical Physics, Cambridge University.

For obtaining values regarding the crosstalk, the parameters from the fitting can be used. The cycle threshold (Ct) is computed as the point equal to half of the second derivative of the resulting fitted function. $C_0$, also known as $C_{INF}$, can represent the symmetry midpoint of the sigmoidal curve. Further, $\Delta_{cycle} = C_{INF} - C_t$.

In the following, the term "receiver" refers to the channel that receives (additional) crosstalk signal (signal being affected by the crosstalk), and the term "donor" refers to the channel that provides or produces crosstalk.

In a preferred embodiment, a linear regression between the adjacent optical channel (donor) of the receiver can be performed for the cycles between Ct and $C_{INF}$ plus $\Delta_{Cycle}$. If $C_{INF}$ plus $\Delta_{Cycle}$ > Max Cycles all the cycles of the amplification can be used.

For each receiver, as many linear regressions can be made as adjacent channels (donors) have been determined. This can be understood as the depth of combinations of channels (donor-receiver) that may be considered to remove the crosstalk. In a preferred embodiment, a maximum of two receivers is considered and the respective crosstalk calculated.

In a preferred embodiment, for each one of the linear regressions a crosstalk value $c_v$ and a goodness $R^2$ can be obtained. The crosstalk value $c_v$ can be the slope between the adjacent channel between the cycles $C_t$ and $C_{INF}$ plus $\Delta_{Cycle}$, from the donor. The $R^2$ can be the goodness of the fit of the signal for the channel.

In a preferred embodiment, additional conditions may indicate whether the crosstalk value is considered valid. Some of possible conditions are as follows:

- Cross talk value $c_v$ > DIN_CROSSTALK_BUND_LOW (minimum boundary defined in the configuration file specifically for each reaction chamber and combination of channels). This boundary is defined as the quantile 2 of a representative sample;

- Cross talk value $c_v$ < DIN_CROSSTALK_BUND_HIGH (maximum boundary defined in the XML configuration file specifically for each reaction chamber and combination of channels). This boundary may be defined as 2.5 times the median of a representative sample; and/or
- $R^2$ > 0.95.

The lower boundary can be set to the 2nd quartile of a representative sample. The upper boundary can be set to two times the median of a representative sample. If the obtained crosstalk value $c_v$ is found valid, the signal of the channel can be corrected in the following way:

$$(receiver\_corrected = receiver - sum(donors*slope)).$$

$$Correction_i = \sum_j Donor_{ij} \times Slope_{Donor_j \rightarrow Receiver}$$

$$Receiver\ Corrected_i = Receiver_i - Correction_i$$

Being i the cycle, as in our preferred embodiment each measurement is associated to a PCR cycle. And, being j the donor, as it can be considered crosstalk produced by multiple donor channels.

In a preferred embodiment, a default value can be used in case the crosstalk value $c_v$ is not valid,. This value can, for example, be defined in the configuration file by DIN_CROSSTALK BUND_DEFAULT, or it can be taken from a separate calibration table (if CALIBRATION_MATRIX DEFAULT_ENABLE is set to 1). Or the default value may be set to the upper Tukey fence value of a representative sample (median + 1.5*IQR). The correction - in case that the crosstalk value cv is not valid - can be performed by the same calculation in which the crosstalk value cv was found valid.

According to a second aspect, a use of signal collection in a process that utilizes fluorescence detection at multiple wavelengths, especially a qPCR process, is provided. According to the second aspect, at least two channels are used in the process, wherein after each signal for the two channels has been recorded for the process, a correlation of the first signal and the second signal is made in view of a crosstalk.

The use is particularly advantageous because the signal measured during the "real" process can be used for crosstalk correlation. No calibration process or calibration measurement has to be performed. Moreover, when considering the signal measured during the "real" process the crosstalk influence can be more reliable considered.

According to a third aspect, a device for performing a process that utilizes fluorescence detection at two or more, i.e. multiple, wavelengths, especially a qPCR process, is provided. The process uses at least two channels. The device comprises: at least a receiving device for receiving a process chamber or reaction vessel holding or containing a respective sample; and at least one optical module capable of emitting and receiving light such that an excitation at a wavelength band and reveving signal at a wavelength band is possible, for example for exciting fluorescent dyes and receiving their fluorescent emission. The optical module can comprise a light or excitation source, at least one optical filter and/or a detector. The optical module may be configurable to excite or receive fluorescence in one or more wavelengths or wavelength bands, for example via exchange of the light source, or exchange of the optical filter/optical filter set, and/or exchange of the detector, or exchange of the module as a whole in relation to each the process chamber. However, it is also possible that for each wavelength an optical module can be provided. The device can further comprise a motor to move or rotate the receiving device, for example a disk or plate, in which the process chamber is situated and/or the optical module to initiate a relative movement between the receiving device and the optical module. As a possibility further/additional process chambers or reaction vessels may be situated in the receiving device. Thus, the optical module can be move relative to reaction vessels such that an automatic measurement of a plurality of samples/probes is possible. The device further comprises a data acquisition device adapted to receive the signal of each of the channels, wherein the data acquisition device is capable of performing the described method of data collection according to the present description.

The device is particularly advantageous because even slight changes in the signals that originate from a crosstalk which may change during measurement can be, especially in an automatic manner, considered.

The invention is described in the present description with reference to a method, a use and an apparatus, the explanations of the said aspects of method, use and apparatus complementing each other. Features of the method can be formulated and supplemented in terms of use or device. An unconstrained combination is possible.

Other objects, features, advantages and aspects of the present application will become apparent to those skilled in the art from the following description and appended claims. It should be understood, however, that the following description, appended claims, and specific examples, while indicating preferred embodiments of the application, are given by way of illustration only.

**BRIEF DESCRIPTION OF THE FIGURES**

**Fig. 1:** Raw data obtained from a (single) reaction chamber of three channels obtained by qPCR as a signal of fluorescence over cycle;

**Fig. 2:** Parameters of the fitting of the raw data of the three channels shown in Fig 1 and the fitted curves of the raw data of the three channels shown in Fig. 1 as a signal of fluorescence over cycle;

**Fig. 3:** Parameters of the linear regression between a channel and a further channel shown in Fig. 1 and a plot of the channel signal over the further channel signal; and

**Fig. 4:** A plot of the raw data shown in Fig. 1 together with signal corrected by the calculated crosstalk.

**DETAILED DESCRIPTION OF THE INVENTION**

The different aspects and embodiments of the invention disclosed herein make important contributions to the art as is also explained in the following.

**THE METHOD ACCORDING TO THE FIRST ASPECT**

According to a first aspect, a method for signal collection in a process that utilizes fluorescence detection at multiple wavelengths, especially a qPCR process, is provided. The method uses at least two channels, wherein for each of the at least two channels an excitation condition and a detection condition is provided for one of the multiple wavelengths, respectively, and wherein during the signal collection in the fluorescence detection: i) a first signal in the first channel is recorded; ii) a second signal in the second channel is recorded; and iii) a crosstalk correlation is determined using a dependency between the first signal and the second signal of the fluorescence detection.

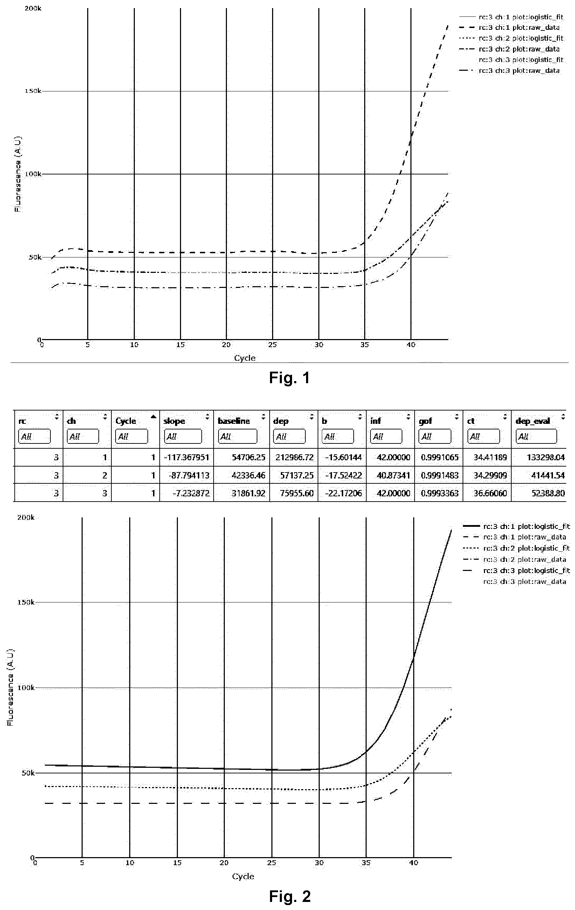

In Fig. 1 raw data for a qPCR process are shown as an example for a process in which fluorescence detection at multiple wavelengths is utilized. The fluorescence signal of three channels has been determined by a data acquisition device over the cycles.

Fig. 2 shows an exemplary curve fitting of the fluorescence signal over the cycles in accordance with the BOBYQA algorithm. Fig. 2 also shows the parameters of the fitting curves, especially parameter Ct (cycle threshold) and $C_{INF}$, given in the table of Fig. 2 as "inf" (symmetry midpoint of the sigmoidal curve).

The fitted curves are filtered such that the curves are considered between Ct and $C_{INF} + \Delta_{cycle}$, wherein $\Delta_{cycle} = C_{INF}$ - Ct.

For considering the crosstalk correlation/correction, channel 1 and channel 3 are considered as donors for the crosstalk and channel 2 is receiver of the crosstalk.

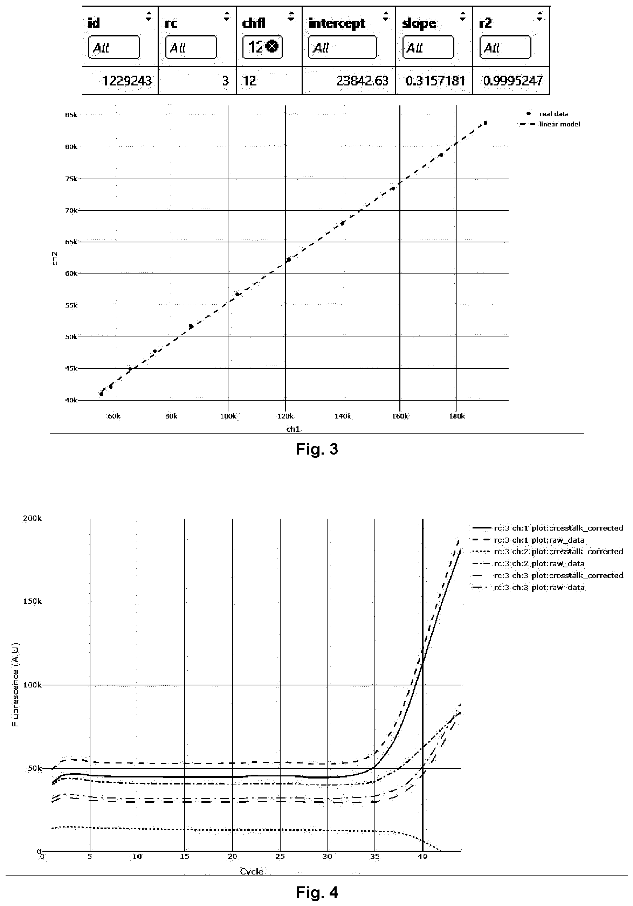

Fig. 3 shows a linear regression carried out between adjacent channels. In Fig. 3 the linear regression is performed between channel 1 and channel 2. Further, a linear regression is performed between channel 3 and channel 2 which is not shown. The slope given in the table of Fig. 3 represents the crosstalk value $c_v$.

The signal for each of the cycles i in the receiver channel is corrected by the signal of the donor channels j multiplied by the crosstalk value $c_v$ obtained by the linear regression between the receiver channel and the respective donor:

$$Correction_i = \sum_j Donor_{ij} \times Slope_{Donor_j \rightarrow Receiver}$$

$$Receiver\ Corrected_i = Receiver_i - Correction_i$$

The corrected signals are shown in Fig. 4.

**THE USE ACCORDING TO THE SECOND ASPECT**

According to a second aspect a use of signal collection in a process that utilizes fluorescence detection at multiple wavelengths is provided. The process can be a qPCR process, wherein in the process at least two channels are used, wherein after each signal for the two channels has been recorded for the process, a correlation of the first signal and the second signal is made in view of a crosstalk.

The use refers to the method described.

**THE DEVICE ACCORDING TO THE THIRD ASPECT**

According to a third aspect a device for performing a process that utilizes fluorescence detection at multiple wavelengths, especially a qPCR process, using at least two channels, is provided. The device comprises a motor to rotate a disk having a plurality of process chambers each holding a respective sample; and at least two optical modules; wherein each of the plurality of optical modules includes an optical channel having a light source selected for excitation of a wavelength and a filter and/or lens to capture fluorescent light emitted at a wavelength; wherein the device further comprises a data acquisition device adapted to receive the signal of each of the channels, wherein the data acquisition device is capable of performing the method described in the present description.

**Claims**

1. Method for signal collection in a process that utilizes fluorescence detection at multiple wavelengths, especially a qPCR process or dPCR process, using at least two channels,

   wherein for each of the at least two channels an excitation condition and a detection condition is provided for one of the multiple wavelengths, respectively, and
   wherein
   during the signal collection in the fluorescence detection:

   i) a first signal in the first channel is recorded;
   ii) a second signal in the second channel is recorded; and
   iii) a crosstalk correlation is determined using a dependency between the first signal and the second signal of the fluorescence detection.

2. Method according to claim 1, wherein a linear dependency between the first signal and the second signal of the process is considered.

3. Method according to claim 1 or 2, wherein a linear regression between the two channels is carried out.

4. Method according to any one of claims 1 to 3, wherein at least one further linear regression is carried out for at least one additional adjacent channel of one of the at least two channels.

5. Method according to claim 3 or 4, wherein for each of the linear regressions a crosstalk value cv depending on the slope of the linear regression and a goodness $R^2$ are obtained.

6. Method according to any one of claims 3 to 5, wherein for it is decided whether a linear regression is considered valid in an automatic supported or an autonomously automatic manner.

7. Method according to claim 6, wherein the consideration whether the linear regression is considered valid is carried out on a decision whether the crosstalk value cv is above a maximum boundary value.

8. Method according to claim 6 or 7, wherein the consideration whether the linear regression is considered valid is carried out on a decision whether the crosstalk value cv is below a minimum boundary value.

9. Method according to any one of claims 6 to 8, wherein the consideration whether the linear regression is considered valid is carried out on a decision whether their goodness $R^2$ is above a certain value.

10. Method according to any one of claims 1 to 9, wherein a correction is performed by subtracting the signal of one of the at least two channels by substantially the slope of a linear regression between the two channels multiplied by the signal of the second channel of the at least two channels.

11. Method according to any one of claims 1 to 10, wherein the at least two channels are adjacent channels.

**12.** Method according to any one of claims 1 to 11, wherein the signal in the two channels is recorded until saturation of the signal is detected.

**13.** Method according to any one of claims 1 to 12, wherein the recorded signal for each of the two channels is fitted.

**14.** Use of signal collection in a process that utilizes fluorescence detection at multiple wavelengths, especially, a qPCR process, wherein in the process at least two channels are used, wherein after each signal for the two channels has been recorded for the process, a correlation of the first signal and the second signal is made in view of a crosstalk.

**15.** Device for performing a process that utilizes fluorescence detection at multiple wavelengths, especially a qPCR process, using at least two channels, the device comprising:

at least a receiving device for receiving a process chamber holding a respective sample; and at least one optical module which is adapted to emit and receive light such that an excitation at a wavelength band and receiving light at a wavelength band is possible, for example for exciting fluorescent dyes and receiving their fluorescent emission;
wherein the device further comprises a data acquisition device adapted to receive the signal of each of the channels, wherein the data acquisition device is capable of performing the method according to any one of claims 1 to 13.

**Fig. 1**

| rc | ch | Cycle | slope | baseline | dep | b | inf | gof | ct | dep_eval |
|----|----|-------|-------|----------|-----|---|-----|-----|----|----------|
| All | All | All | All | All | All | All | All | All | All | All |
| 3 | 1 | 1 | -117.367951 | 54706.25 | 212986.72 | -15.60144 | 42.00000 | 0.9991065 | 34.41189 | 133298.04 |
| 3 | 2 | 1 | -87.794113 | 42336.46 | 57137.25 | -17.52422 | 40.87341 | 0.9991483 | 34.29909 | 41441.54 |
| 3 | 3 | 1 | -7.232872 | 31861.92 | 75955.60 | -22.17206 | 42.00000 | 0.9993363 | 36.66060 | 52388.80 |

**Fig. 2**

| id | rc | chfl | intercept | slope | r2 |
|---|---|---|---|---|---|
| *All* | *All* | 12 ⊗ | *All* | *All* | *All* |
| 1229243 | 3 | 12 | 23842.63 | 0.3157181 | 0.9995247 |

**Fig. 3**

**Fig. 4**

## EUROPEAN SEARCH REPORT

**Application Number**

EP 23 18 1242

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/171677 A1 (LUDOWISE PETER D [US]) 5 July 2012 (2012-07-05) * paragraphs [0007]-[0008], [0010]-[0012], [0016], [0056], [0079], [0187], [0219]; figure 1 * | 1-15 | INV. C12Q1/6818 C12Q1/6851 G01F11/00 G01N21/00 |
| X | WO 2009/003645 A1 (ROCHE DIAGNOSTICS GMBH [DE]; HOFFMANN LA ROCHE [CH] ET AL.) 8 January 2009 (2009-01-08) * page 2, lines 25-30; page 9, lines 15-17; page 11, lines 8-16; page 11, lines 20-27 * | 1-15 | |

**TECHNICAL FIELDS SEARCHED (IPC)**

G01F
G01N
C12Q

The present search report has been drawn up for all claims

| Place of search | Date of completion of the search | Examiner |
|---|---|---|
| Munich | 8 December 2023 | Celler, Jakub |

## ANNEX TO THE EUROPEAN SEARCH REPORT
## ON EUROPEAN PATENT APPLICATION NO.

EP 23 18 1242

This annex lists the patent family members relating to the patent documents cited in the above-mentioned European search report.
The members are as contained in the European Patent Office EDP file on
The European Patent Office is in no way liable for these particulars which are merely given for the purpose of information.

08-12-2023

| Patent document cited in search report | Publication date | Patent family member(s) | | Publication date |
|---|---|---|---|---|
| US 2012171677 A1 | 05-07-2012 | CN | 102482720 A | 30-05-2012 |
| | | DK | 2475788 T3 | 30-07-2018 |
| | | DK | 3375890 T3 | 14-04-2020 |
| | | DK | 3375891 T3 | 27-04-2020 |
| | | EP | 2475788 A1 | 18-07-2012 |
| | | EP | 3375890 A1 | 19-09-2018 |
| | | EP | 3375891 A1 | 19-09-2018 |
| | | ES | 2680495 T3 | 07-09-2018 |
| | | ES | 2785672 T3 | 07-10-2020 |
| | | ES | 2791369 T3 | 04-11-2020 |
| | | MX | 352971 B | 15-12-2017 |
| | | PT | 2475788 T | 24-07-2018 |
| | | PT | 3375890 T | 23-04-2020 |
| | | PT | 3375891 T | 11-05-2020 |
| | | US | 2012171677 A1 | 05-07-2012 |
| | | US | 2018201987 A1 | 19-07-2018 |
| | | WO | 2011031585 A1 | 17-03-2011 |
| WO 2009003645 A1 | 08-01-2009 | AT | E518966 T1 | 15-08-2011 |
| | | CA | 2691907 A1 | 08-01-2009 |
| | | CN | 101688841 A | 31-03-2010 |
| | | EP | 2160594 A1 | 10-03-2010 |
| | | ES | 2370678 T3 | 21-12-2011 |
| | | HK | 1140261 A1 | 08-10-2010 |
| | | JP | 5400768 B2 | 29-01-2014 |
| | | JP | 2010531649 A | 30-09-2010 |
| | | US | 2009035779 A1 | 05-02-2009 |
| | | WO | 2009003645 A1 | 08-01-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20180201987 A1

**Non-patent literature cited in the description**

- **POWELL, M. J. D.** The BOBYQA algorithm for bound constrained optimization without derivatives (Report). Department of Applied Mathematics and Theoretical Physics, Cambridge University, June 2009